# EUROPEAN PATENT APPLICATION

(11) **EP 2 679 218 A1**
(43) Date of publication of application: **01.01.2014**
(21) Application number: 13185616.3
(22) Date of filing: 26.03.2010
(51) Int. Cl.: A61K 9/00, A61K 47/36

(54) **Moisturising Composition**

(30) Priority: 01.04.2009 GB 0905677
(62) Divisional of application: 10714652.4
(71) Applicant: Yes Syzygy Limited, Hampshire GU34 3NS (GB)
(72) Inventor: Lennox, Susan Mary, Alton, Hampshire GU34 3NS (GB); Brooks, Sarah Annabelle, Petersfield, Hampshire GU32 2DJ (GB)
(74) Representative: Brooks, Nigel Samuel

(57) **Abstract**

A composition which provides moisturisation and lubricity. It has a pH of 3.0 to 5.5; and has an osmolality of 100 to 400 mosm. The composition acts moisturiser for love making. It also has a viscosity of 12,000 to 30,000 centipoise.

## Description

The present invention relates to a moisturising composition.

Couples can experience discomfort when making love. Often this is caused by vaginal dryness.

In International Patent Application No. WO 2006/092581 there is described and claimed - at least on entry into the UK regional phase No GB 2,437,903 A - A lubricating composition comprising:
- linseed extract and a further seed polysaccharide extract soluble in water,
- a pH buffer and
- at least one preservative for prevention of growth of micro-organisms.

In production, this composition has been acidic. Vaginal fluids are acidic typically in the region of a pH of 4 to 5. However sperm can be incapacitated by acidity, in that they cease to swim, i.e. become immotile. Sperm can also lose viability if in an aqueous environment where the osmolality is either too high, hyper-osmotic (greater than 380 mosm) or too low hypo-osmotic (less than 280 mosm). Semen is normally has a pH between 7 & 8 and has an osmolality in the region of 320 mosm, ranging from 260 to 365 mosm.

Vaginal acidity is advantageous in discouraging infections such as thrush (Candida albicans), Bacterial Vaginosis and sexually transmitted infections such as Trichomonas. These infections reduce ability to conceive.

There is a correlation between vaginal dryness and infertility.

We have sought to develop a lubricating composition to help men and women, who are trying to conceive, whether or not they are suffering from infertility. However the fundamental dichotomy of vaginal acidity not suiting the sensibilities of sperm remains.

In seeking to overcome the dichotomy, we have devised and invented a dual composition lubricant, one composition being sperm friendly and the other composition being vagina friendly.

However, one of these compositions can be used alone for providing lubrication during love making when not trying to conceive.

The object of the present invention is to provide a moisturising and lubricating composition.

According to the invention there is provided a composition that:
- provides moisturisation;
- provides lubricity;
- has a is slightly to moderately acidic; and
- has an osmolality of 100 to 400 mosm
   when used in an intimate aqueous moisturising and lubricating composition.

By providing "lubricity" is meant providing lubrication in the manner that soapy water lubricates wet hands rubbed together which can then slip easily across each other.

By providing "moisturisation" is meant ability to transport moisture to human tissue.

Preferably the composition is substantially iso-osmotic with typical vaginal osmolality.

Whilst we can envisage that the composition could be formulated with synthetic materials, such as hydroxyethylcellulose, we prefer to formulate it with naturally derived materials, i.e. materials which have not been chemically synthesised, at least as far as possible. Not least, we prefer to use organically derived materials, i.e. materials derived from plants and preferably plants not treated with synthetic chemicals, because they are less likely in our experience to contain impurities such as traces of solvents and catalysts used in their product. For this reason, we further prefer to use organically derived materials that have not been extracted utilising synthetic materials such as extraction solvents, for instance hexane. For instance where we use aloe vera, we prefer to use it dried as opposed to extracted. In particular we prefer to formulate the composition with either or both of linseed and a further plant polysaccharide.

Normally we would expect the composition to be hydrophilic; however, for lubricity, we can envisage composition incorporating emulsified organically derived hydrophobic constituents, namely beeswax, plant oils and butters. Nevertheless we prefer to avoid linseed oil as such on account of its odour on oxidisation.

By careful choice of the water source from which the composition is prepared, the composition may be mildly acidic without addition. If this cannot be achieved by use of hard or soft water, we prefer to use citric acid, lactic acid or hydrochloric acid if the respective composition is too alkaline or sodium or potassium hydroxide if too acid. Other common acids and alkalis are possible. To maintain acidity, we prefer to incorporate pH buffers in the composition, such as phosphates.

Again by choice of the water and other substances, the composition is expected to be substantially iso-osmotic to the vaginal osmolality. If the osmolality requires to be adjusted, this may be achieved by use of an organic solute such as a sugar or an inorganic salt such as sodium chloride.

Preferably the composition will have a pH of 3.0 to 5.5 - more likely 3.0 to 4.5 - and an osmolality of 100 to 400 mosm, typically 250-310 mosm. Further we believe that a target viscosity for this composition is 12,000 to 30,000 centipoise. Nevertheless, we aim for the moisturising composition to be a compromise in being a moisturiser and a lubricant for love making at any time.

We envisage that we can include lactobacillus in the moisturising composition to enhance it natural occurrence in the vagina.

Additionally we prefer to incorporate a muco-adherent in the moisturising composition, in the interest of moisturisation.

To help understanding of the invention, a specific embodiment thereof will now be described by way of example and with reference to the accompanying drawings, in which:

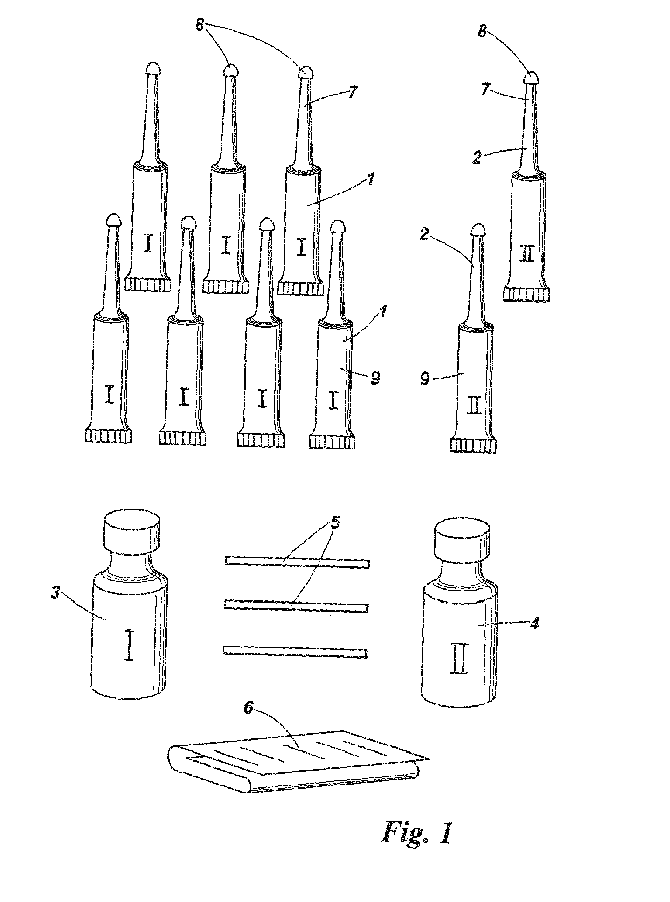

Figure 1 is a diagram of an intimate lubrication kit in accordance with the invention.

Referring to the drawing, the intimate lubrication kit there shown has seven applicators 1 filled with lubricant and three applicators 2 filled with moisturiser. In addition, a bottle 3 of the lubricant and a bottle 4 of the moisturiser are provided. The kit also includes ovulation test strips 5 and instructions 6.

The applicators 1,2 have long thin necks 7 with closure caps 8 and integral squeezable containers 9. The necks are typically 50mm long and the containers typically have a 5ml capacity. The bottles have 25ml capacity.

The compositions are prepared in like manner to that described in International Patent Application No. WO 2006/092581 from a common aqueous mixture of linseed extract and Xanthan gum, with small quantities of lonicera caprifolium extract and/or potassium sorbate as preservatives.

The composition has citric acid added to adjust its pH to 3.0 to 4.5 and its osmolality to 250-310 mosm. Further it has aloe vera extract added for its tissue benefits.

We believe the composition is vagina friendly.

The instructions for use are as follows:

### Intimate Lubrication Kit

*This kit is for couples experiencing difficulty in making love, in particular because of vaginal dryness. It may assist in conception.*

*The kit comprises 7 applicators of lubricating composition for use during the days up to and including ovulation and 3 applicators of a slightly thicker composition for use just after ovulation.*

*The lubricating composition is neutral or slightly acid with a view to being sperm friendly. The thicker composition is slightly acid to mirror vaginal acidity and includes a moisturiser for vaginal friendliness. It is also a lubricant.*

*The lubricating composition is applied to a couple's intimate organs when making love during the woman's most fertile period, i.e. typically the six days up to and including ovulation. Conveniently the composition can be applied to her vagina using the long necked applicators. The moisturising composition is applied to her vagina at least 24hrs after ovulation is expected to have occurred.*

*Ovulation test strips are provided for assistance in determining the timing of ovulation.*

*Some couples may find it more comfortable to apply additional lubricant externally, either from one of the long necked applicators or from a separately provided bottle. Similarly, the moisturiser may be applied externally from the second long necked container or the bottle of moisturiser.*

According to the present invention the moisturiser can be used as a moisturiser and lubricant for making love when conception is not the aim.

The invention is not intended to be restricted to the details of the above-described embodiment. For instance, other aqueous polysaccharide aqueous extracts, for instance, Yellow Mustard seed gum, Acacia gum, gums from seaweeds (eg Alginates or Carrageenan), β-Glucans (derived from barley, oats, rye and wheat) and fruit gums. Preferred additional gums are Galactomannan gums, since they exhibit a synergistic phenomena resulting from their Galactomannan structures of long smooth sections of their molecules, for example Locust Bean gum, Guar gum, Cassia gum, Tara Gum, and Konjac Mannan gel which is a Glucomannan and is stereochemically similar to a Galactomannan. Certain of these gums are prepared in the manner of the linseed extract, for instance Yellow Mustard seed gum. Xanthan gum is prepared by action of the Xanthomonas campestris on maize seed. Further in place of potassium sorbate, Citricidal and/or phenoxyethanol may be used as preservatives

Whilst we prefer to use naturally occurring substances, we can envisage use of synthetic polysaccharides, Hydroxyethyl cellulose, glycerine, polyethylene oxide, polycarbophil, carbomers and / or glycols.

## Claims

1. A composition that:
• provides moisturisation;
• provides lubricity;
• is slightly to moderately acidic; and
• has an osmolality of 100 to 400 mosm
when used in an intimate aqueous moisturising and lubricating composition.

2. A composition as claimed in claim 1, wherein the composition is substantially iso-osmotic with vaginal osmolality.

3. A composition as claimed in claim 1 or claim 2 wherein the osmolality is 250-310 mosm.

4. A composition as claimed in claim 1, claim 2 or claim 3, wherein the pH is 3.0 to 5.5 and preferably 3.0 to 4.5.

5. A composition as claimed in any preceding claim, wherein the composition is partially or totally formulated with synthetic constituents.

6. A composition as claimed in any one of claims 1 to 4, wherein the composition is partially or totally formulated with organically derived constituents.

7. A composition as claimed in claim 6, wherein at least some of the organically derived constituents have been extracted without utilising synthetic materials.

8. A composition as claimed in claim 6, wherein at least one of the organically derived constituents has been dried.

9. A composition as claimed in any preceding claim, wherein the composition contains either or both of an aqueous extract of linseed (flax) and a further seed polysaccharide.

10. A composition as claimed in any preceding claim, wherein the composition is hydrophobic.

11. A composition as claimed in any preceding claim, wherein the pH of the composition derives from water used in its preparation.

12. A composition as claimed in anyone of claims 1 to 10, wherein the pH of the composition is controlled by addition of selected ones of citric acid, lactic acid, hydrochloric acid, sodium hydroxide and potassium hydroxide.

13. A composition as claimed in any preceding claim, wherein the composition contain pH buffers and/or contains an organic solute or an inorganic salt for control of osmolality.

14. A composition as claimed in any preceding claim, wherein the composition has a viscosity of 12,000 to 30,000 centipoise, and/or has non-Newtonian properties.

15. A composition as claimed in any preceding claim, wherein the moisturising composition contains a muco-adherent, and/or contains preservatives such as potassium sorbate and/or contains lactobacillus.
